(19) **Europäisches Patentamt / European Patent Office / Office européen des brevets**

(11) **EP 3 291 795 B1**

(12) **EUROPEAN PATENT SPECIFICATION**

(45) Date of publication and mention of the grant of the patent:
**07.08.2019 Bulletin 2019/32**

(51) Int Cl.:
*A61K 9/107* (2006.01)     *A61K 9/19* (2006.01)
*A61K 9/51* (2006.01)

(21) Application number: **16721502.9**

(86) International application number:
**PCT/IB2016/052544**

(22) Date of filing: **04.05.2016**

(87) International publication number:
**WO 2016/178158 (10.11.2016 Gazette 2016/45)**

(54) **METHOD FOR ENCAPSULATING PHARMACEUTICAL ACTIVES**

VERFAHREN ZUR VERKAPSELUNG VON PHARMAZEUTISCHEN WIRKSTOFFEN

PROCÉDÉ D'ENCAPSULATION DE PRINCIPES ACTIFS PHARMACEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.05.2015 ZA 201503116**

(43) Date of publication of application:
**14.03.2018 Bulletin 2018/11**

(73) Proprietor: **CSIR**
**0002 Pretoria (ZA)**

(72) Inventor: **ADELEKE, Oluwatoyin Ayotomilola**
**Belleville, New Jersey 07109 (US)**

(74) Representative: **Moore, Michael Richard et al**
**Keltie LLP**
**No.1 London Bridge**
**London SE1 9BA (GB)**

(56) References cited:
**EP-A1- 2 082 750     WO-A1-92/17162**

- **DAN YANG YING ET AL: "Microencapsulated Lactobacillus rhamnosus GG Powders: Relationship of Powder Physical Properties to Probiotic Survival during Storage", JOURNAL OF FOOD SCIENCE, vol. 75, no. 9, November 2010 (2010-11), pages E588-E595, XP055024657, ISSN: 0022-1147, DOI: 10.1111/j.1750-3841.2010.01838.x**
- **TOORISAKA E ET AL: "An enteric-coated dry emulsion formulation for oral insulin delivery", JOURNAL OF CONTROLLED RELEASE, ELSEVIER, AMSTERDAM, NL, vol. 107, no. 1, 20 September 2005 (2005-09-20), pages 91-96, XP027664154, ISSN: 0168-3659 [retrieved on 2005-09-20]**

## Description

This invention relates to the encapsulation of pharmaceutical actives. In particular, the invention relates to a method for encapsulating a pharmaceutical active. The method is capable of producing pharmaceutical active-loaded particles (i.e. drug-loaded particles) suitable for the production of nano and/or micro pharmaceutical formulations.

### Field of the Invention

This present invention thus lies in the field of drug delivery/pharmaceutical formulations and particularly relates to a distinctive encapsulation method or technique that can be described as an oleo-polymeric method for encapsulating pharmaceutical actives or drugs with either hydrophilic or hydrophobic properties. The oleo-polymeric method of the invention advantageously can produce nano/micro pharmaceutical formulations that can be administered via the oral or transmucosal routes.

### Background to the Invention

Encapsulation is widely applied in pharmaceutical formulation production for entrapping essential or active ingredients into an external carrier system in order to impart protection against oxidation, degradation and isomerization as well as enhance stability, improve functionality and extend product shelf life. Furthermore, encapsulation of a bioactive can be utilized for controlling the release of bioactives under both *in vitro* and *in vivo* conditions. Nano/micro encapsulation or fabrication refers to the tools and methods that can be used for modifying the structures and properties of a substance/material at the nano scale (< 1000 nm) or micro scale (< 100 $\mu$m).

In this specification, the term "pharmaceutical active" is intended to refer to an active pharmaceutical ingredient, also known as an API.

Advancements in the production of nanomedicines possess great potential for reforming pharmacotherapeutical processes. Nano/micro-range drug delivery systems constitute an important part of nanomedicine. The development of useful pharmaceuticals based on the principles of nanomedicine can be described as the science and technology of composite systems within the nanometer scale (10-1000 nm) consisting of two or more constituents which are the drug molecule(s) and excipient(s). A nano-formulation consisting of only the pulverized pharmaceutically active molecule is also possible. Defining nanotechnology in drug delivery by placing limits on size is not always helpful because the usefulness of such systems is typically not based on a size limit. In a nutshell, useful drug delivery systems span over accurately nano-sized (< 1000 nm) to micro-sized systems ($\leq$ 100 $\mu$m) and have been useful for developing clinically effective formulations. From a real-world perspective, based on these reasons, nanotechnology includes microtechnology and nanofabrication or nanomanufacturing and its micro counterparts.

Research has proven that nano/micro encapsulation is an ideal way of preparing controlled, rate-modulated pharmaceutical formulations that are beneficial to patients. Nano/micro formulations have numerous unique advantages which include but are not limited to the following: (i) delivery of drug molecules with poor solubility and permeability, (ii) attainment of constant plasma drug concentration and increased relative bioavailability, (iii) site-specific targeted drug delivery, (iii) immediate release properties for rapid onset of pharmacological action, (iv) formulation taste improvement, (v) sustained release leading to reduction of dosage frequency as well as both local and systemic side effects, (vi) enhanced mucoadhesion and transmucosal permeation (vii) good stability suitable for extended storage duration with little or no loss of drug activity, (viii) enables administration of formulations (e.g. effervescent dosage forms) to patients who are unable to chew or swallow, (ix) increased formulation morphological flexibility and surface area; (x) less dose of active drug is required, (xi) decreased fasted or fed erraticism and (xii) reduced patient-to-patient variability.

Several methods have been developed for the preparation of a variety of polymeric nano/micro-products. This can be divided into two main groups namely dispersion of preformed polymers and polymerization of monomeric units. The dispersion of preformed polymers encompasses processes such as evaporation or extraction of solvent, nanoprecipitation, emulsification/solvent diffusion, salting out, dialysis, supercritical fluid technology and hot melt encapsulation. Preparation methods which involve monomeric units include techniques such as emulsion, mini/micro emulsion, interfacial, surfactant-free emulsion, controlled/living radical polymerizations and interfacial polycondensation.

Currently, the emulsion technique, either using monomers or preformed polymers dispersed in a mixed aqueous and/or non-aqueous continuous phase, is one of the quickest, easily scalable, most commonly employed methods of preparing nano/micro formulations. It presents several advantages such as batch-to-batch reproducibility, simplicity and narrow size distribution as well as relatively high encapsulation efficiency. Its major disadvantages include the use of toxic organic solvents, harsh/varying temperature, pressure and pH conditions as well as leakage of encapsulated drug molecules, especially hydrophilic-natured ones, into the aqueous-organic external phase thereby reducing encapsulation efficiency/drug loading capacity. In other words, this method is more suitable for hydrophobic drugs indicating its non-versatility.

The vast advantages of nano/micro formulations and the demerits of the choice of emulsion technique make developing a unique technology which can produce pharmaceutically useful nano/micro formulations for commercialization processes and which overcomes the drawbacks of the prior art desirable.

WO 92/17162 A1 discloses a method for preparing a lyophilized oil-in-water emulsion which comprises freezing and lyophilizing the emulsion in a single cycle. The oil-in-water emulsion comprises of a lipid or oil (medium chain triglycerides, linoleic acids, and vegetable oil) suitable for injection, an emulsifier/surfactant (egg lecithin or combination of egg lecithin and the non-ionic surfactant Pluronic F 68), agents to improve isotonicity (glycerin), a carbohydrate to provide bulk (lactose), preservatives (methylparaben) or anti-oxidants ($\alpha$-tocopherol) and water. Lipophilic pharmaceutically active ingredients/drugs are incorporated into the oil/lipid phase of the emulsion. The oil-in-water emulsion is prepared at 50-55°C by combining water and lactose and dispersing egg lecithin in the aqueous phase followed by homogenization. Glycerin, the isotonic agent, is added by stirring in and water is added as needed. The lyophilisation process involves subjecting the emulsion to a series of temperature gradients by cooling the emulsion at -40°C for 0-24 hours; slowly raising temperature to +15°C over 12 hours (24-36 hours), holding temperature at +15°C for 12 hours (36-48 hours) and raising temperature to +30°C for 24 hours (48-72 hours). The lyophilized product is then removed from the lyophilizer at 72 hours. The lyophilized product is suitable for reconstitution in sterile water for parenteral administration and the mean particle size of the product is approximately 340 nm.

Dan Yang Ying et al: "Microencapsulated Lactobacillus rhamnosus GG Powders: Relationship of Powder Physical Properties to Probiotic Survival during Storage", Journal of Food Science, vol. 75, no.9, November 2010, proposes the encapsulation of a freeze-dried commercial *Lactobacillus rhamnosus* in an emulsion-based formulation stabilized with whey protein and resistant starch processed through spray drying or freeze drying to produce probiotic microcapsules. For the freeze drying process, the emulsion was frozen overnight at - 18°C, then freeze dried for 48 hours and milled under aseptic conditions. Produced microcapsules had irregular and broken morphologies with particle sizes ranging between 10 and 5000 $\mu$m.

Toorisaka E et al: "An enteric-coated dry emulsion formulation for oral insulin delivery", Journal of Controlled Release, Elsevier, Amsterdam, NL, vol. 107, no. 1, 20 September 2005 describes the development of an enteric-coated dry emulsion formulation for oral insulin delivery. In order to accomplish this objective, an aqueous solution of fluorescein-isothiocyanate-insulin (FITC-INS) in 0.01 M hydrochloric acid and hexane solution containing a hydrophobic surfactant, namely sucrose erucic ester, was mixed with a homogenizer at 26,000rpm to form a water-in-oil emulsion which was lyophilized for 24 hours. The resultant surfactant coated FITC-INS solid was then readily dispersed into soybean oil to form a solid-in-oil suspension. Thereafter, a solid-in-oil-in-water emulsion was obtained by homogenizing a water-in-oil solution containing hydroxypropylmethylcellulose phthalate (HPMCP), polyoxyethylene hydrogenated castor oil and glycerin with the solid-in-oil suspension at 15,000 rpm. The size of the oil droplets was controlled by extruding the solid-in-oil-in-water emulsion through a Shirasu porous glass membrane and the resultant microspheres were prepared by extruding the emulsion dropwise into 0.05 M hydrochloric acid solution containing sucrose lauric ester and glycerine. The dry emulsion was then prepared by lyophilizing the microparticulates to produce a dry emulsion with an average diameter of 50 $\mu$m.

EP 2082750 A1 describes a method of preparing a degradable slow-releasing microsphere-encapsulated oral vaccine containing enterotoxigenic recombinant protein from *Escherichia coli* for the immunoprophylaxis and treatment of human *Helicobacter pylori* infection. The recombinant protein was evenly mixed with a 2% sodium alginate solution under room temperature followed by the addition of a vegetable oil in a ratio of 2:8 and emulsified at 8,000 r/min for 10 minutes. Subsequently, a $CaCl_2$ solution as cross-linking agent was added by reverse titration to form an oil-in-water emulsion which was centrifuged, washed and resuspended in a chitosan solution for re-encapsulation to obtain a chitosan-sodium alginate double encapsulated protein microsphere solution which was slowly poured into vials, pre-frozen in a refrigerator at -40°C for 12 hours, and directly placed in a pre-chilled vacuum drying machine for drying, slowly raising the temperature to rapidly sublimate water. 8% Mannitol and 0.05% EDTA-$Na_2$ were used as excipient and stabilizing agent for the freeze drying process respectively. The freeze-dried microspheric powder had an average particle size of 3.33 $\mu$m.

According to the invention, there is provided a method for encapsulating a pharmaceutical active, the method including

dispersing a hydrophilic polymeric matrix former into a water phase and a hydrophobic polymeric matrix former into an oil phase, with the pharmaceutical active being present in at least one of the oil phase and the water phase and with the oil phase comprising a vegetable-based edible oil;

forming an emulsion from the oil phase and the water phase; and

lyophilizing the emulsion to form a solid lyophilisate, wherein lyophilizing the emulsion includes first snap freezing the emulsion by cooling the emulsion to a freezing temperature below the temperature at which the emulsion is lyophilized.

Preferably, the emulsion is a finely dispersed emulsion. More preferably, the emulsion is so finely dispersed that it appears mono-phased and homogenous, so that it can be referred to as a nano/micro-emulsion.

The method typically includes comminuting the solid lyophilisate to provide particles or a powder of the encapsulated pharmaceutical active. Preferably, the powder is suitable for the production of nano and/or micro pharmaceutical formulations. The invention thus also extends to a method of producing nano and/or micro pharmaceutical formulations.

Comminuting the solid lyophilisate may include dry milling. Preferably, a free flowing powder is provided by comminution. Preferably, particles of the powder are spherical. The powder is typically a powder of a semi-crystalline solid material.

The hydrophilic polymeric matrix former may be selected from the group consisting of a polyethylene glycol, polyacrylic acid, polyether, polyacrylamide, polyvinyl alcohol, polyethylene oxide, polyvinylpyrrolidone, polyoxazoline, polymethacrylate, polyethylenimine, polyphosphate based polymer and mixtures of two or more thereof.

The hydrophobic matrix former may be selected from the group consisting of a cellulose ether, a poly (lactide-co-glycolide) based polymer, and mixtures of two or more thereof.

In an exemplified embodiment of the invention, the hydrophilic matrix former is polyethylene glycol and the hydrophobic matrix former is ethyl cellulose.

The process may include employing a water soluble polymer as a surfactant or emulsifying agent for the forming of an emulsion, preferably finely dispersed emulsion, from the oil phase and the water phase. In one embodiment of the invention, the water soluble polymer is added to the water phase.

The water soluble polymer may be selected from the group consisting of polyvinyl alcohols, methylcellulose derivatives, cross linked copolymers of acrylic acid, and mixtures of two or more thereof.

In an exemplified embodiment of the invention, the water soluble polymer is polyvinyl alcohol.

The method may include employing a cryoprotectant or lyoprotectant in the emulsion. In one embodiment of the invention, the cryoprotectant or lyoprotectant is added to the water phase.

The cryoprotectant or lyoprotectant may be selected from the group consisting of a monosaccharide or a disaccharide, and mixtures thereof.

Preferably, the cryoprotectant or lyoprotectant is one or more of sucrose, lactose, fructose, mannitol, trehalose, glucose and ribose.

In an exemplified embodiment of the invention, the cryoprotectant or lyoprotectant is a disaccharide in the form of D-fructose.

The vegetable-based edible oil may be selected from the group consisting of coconut oil, peanut oil, soya bean oil, olive oil, corn oil, apricot oil, castor oil, macadamia oil, cottonseed oil, palm oil, rapeseed oil, safflower oil, sunflower oil, almond oil, hazelnut oil, flaxseed oil, grapeseed oil, sesame oil, rice bran oil, mustard oil and mixtures of two or more thereof. In exemplified embodiments of the invention, the vegetable-based edible oil is coconut oil, peanut oil, soya bean oil, olive oil or corn oil.

The method of the invention may thus be characterized by the absence of organic or inorganic chemically synthesized solvents or oils.

The pharmaceutical active may be water soluble or hydrophilic. The water soluble or hydrophilic pharmaceutical active, e.g. isoniazid, may be present in the water phase prior to forming of the emulsion.

The pharmaceutical active may be water-insoluble or hydrophobic. The water-insoluble or hydrophobic pharmaceutical active, e.g. rifampicin, may be present in the oil phase prior to forming of the emulsion.

More than one pharmaceutical active may be encapsulated. One or more pharmaceutical active may be water soluble or hydrophilic. One or more pharmaceutical active may be water soluble or hydrophilic.

Forming an emulsion from the oil phase and the water phase may include vigorously emulsifying the oil phase and the water phase together. In one embodiment of the invention, a blender is used to emulsify the oil phase and the water phase together, with the blender operating at a rotational speed of about 4,000 to about 8,000 rpm.

The water of the water phase may be deionised water.

The emulsion may be lyophilized at a temperature of between about -50°C and about -70°C, preferably between about -55°C and about -70°C, more preferably between about -60°C and about -70°C, e.g. about -60°C.

The emulsion may be lyophilized at a pressure of between about 7.5 Pa(absolute) and about 33.5 Pa(absolute), preferably between about 10.5 Pa(absolute) and about 25.5 Pa(absolute), more preferably between about 1.2.0 Pa(absolute) and about 20 Pa(absolute), e.g. about 16.5 Pa(absolute).

The emulsion may be lyophilized for a period of between about 24 hours and about 120 hours, preferably between about 48 hours and about 120 hours, more preferably between about 72 hours and about 120 hours, e.g. about 96 hours.

Snap freezing the emulsion by cooling the emulsion to a freezing temperature below the temperature at which the emulsion is lyophilized is typically over a freezing period of at least a few minutes.

The freezing temperature may be between about -60°C and about -196°C, preferably between about -100°C and about -196°C, e.g. about -190°C.

The freezing period may be between about 5 minutes and about 60 minutes, preferably between about 10 minutes and about 40 minutes, more preferably between about 20 minutes and about 40 minutes, e.g. about 30 minutes.

The particles of the encapsulated pharmaceutical active or the powder may have an average particle size falling in the nano range. When the particles or powder has an average particle size falling in the nano range, the average particle size may be between about 100 nm and about 999 nm, preferably between about 400 nm and about 950 nm, more preferably between about 400 nm and about 500 nm, e.g. about 400 nm.

Instead, the particles of the encapsulated pharmaceutical active or the powder may have an average particle size falling in the micro range. When the particles of the encapsulated pharmaceutical active or the powder has an average particle size falling in the micro range, the average particle size may be between about 1 $\mu$m and about 5 $\mu$m, preferably between about 1.02 $\mu$m and about 1.62 $\mu$m, e.g. about 1.05 $\mu$m.

The matrix former and the vegetable-based oil and the pharmaceutical active may be selected such that the powder of the encapsulated pharmaceutical active has a dispersity $Đ_m$ (i.e. based on molecular mass) or polydispersity index from dynamic light scattering less than 0.95, preferably less than 0.9, more preferably less than 0.6, even more preferably less than 0.5, still more preferably less than 0.4, e.g. between about 0.10 and about 0.9, or between about 0.20 and about 0.90, or between about 0.30 and about 0.6, or between about 0.3 and about 0.5, or between about 0.3 and about 0.4, e.g. about 0.30.

The matrix former and the vegetable-based oil and the pharmaceutical active may be selected such that a colloidal dispersion of the particles of the encapsulated pharmaceutical active or powder in an aqueous medium may has a zeta potential of at least negative 20.00 mV, preferably between about -20.00 mV and about -50.00 mV, more preferably between about -23.50 mV and about -46.70 mV, most preferably between about -32.90 mV and about -46.30 mV, e.g. about -40.00 mV.

The process may show a product yield of at least about 87%, preferably at least about 95%, more preferably at least about 99%, where the product yield (in percentage) =

$$\frac{\text{total weight lyophilisate}}{\text{total weight of all ingredients}} \times 100.$$

The lyophilisate may have a drug loading of at least about 70%, preferably at least about 98%, more preferably at least about 99%, where the drug loading is the mass percentage of the pharmaceutical active in the lyophilisate.

The invention extends to a lyophilisate and to a powder produced by the method as hereinbefore described.

The lyophilisate may be as hereinbefore described.

The powder may be as hereinbefore described.

In one embodiment of the invention, the powder produced by the method as hereinbefore described has a dispersity $Đ_m$ or polydispersity index from dynamic light scattering of less than 0.95, with a colloidal dispersion of the powder in an aqueous medium having a zeta potential of at least negative 20.00 mV.

As set out hereinbefore, the powder produced by the method as hereinbefore described may have an average particle size falling in the nano range, or an average particle size falling in the micro range.

The invention also extends to a powder as hereinbefore described, and to a pharmaceutical composition or formulation which includes a powder as hereinbefore described.

In particular, the invention extends to a pharmaceutical product which includes a powder produced by the method of the invention, the powder having a dispersity $Đ_m$ or polydispersity index from dynamic light scattering of less than 0.95, with a colloidal dispersion of the powder in an aqueous medium having a zeta potential of at least negative 20.00 mV.

Said powder may have an average particle size falling in the nano range, or an average particle size falling in the micro range.

The powder of the pharmaceutical product may be as hereinbefore described.

The invention will now be described using the following experiments and experimental results and by way of the accompanying diagrammatic drawings in which

Figure 1 is an illustration of one embodiment of a direct dispersion emulsification method or technique in accordance with the invention for producing nano/micro formulations;

Figure 2 is an illustration showing the (a) drug release profile, (b) transmission electron microscopic image (magnification = x 25,000), (c) powder XRD diffractogram and (d) FTIR spectra of an isoniazid-loaded optimized nano/micro formulation prepared in accordance with the invention;

Figure 3 is an illustration showing the (a) transmission electron microscopic image (magnification = x 25,000) (b) drug release profile and (c) powder XRD diffractogram of a rifampicin-loaded optimized nano/micro formulation prepared in accordance with the invention.

## Experimental materials

Polyethylene glycol 4000, poly (vinyl alcohol) 87% - 89% hydrolysed, coconut oil obtained from *Cocos nucifera*, peanut oil, soybean oil, olive oil, corn oil, ethyl cellulose - viscosity 22 cP (Ethocel® 22), isoniazid (hydrophilic; Biopharmaceutical Classifications System (BCS) class borderline I/III) and rifampicin (hydrophobic; BCS class II) were purchased from Sigma Chemical Company (St. Louis, United States of America). D-fructose was obtained from Merck Chemicals (Darmstadt, Germany). All other reagents utilized were of acceptable analytical grade and were used as obtained.

**Preparation of a nano/micro formulation employing a direct dispersion emulsification technique in accordance with the Box-Behnken statistical design**

15 variant nano/micro formulations were prepared utilizing a direct dispersion emulsification technique coupled with lyophilisation and dry milling guided through a 3-level, 3-factor and 3-centre points Box-Behnken quadratic design using Minitab Statistical Software, Version 16 (Minitab Inc., State College, PA, United States of America). Three sets of independent variables classified as the water based phase, oil based phase and homogenization velocity were utilized in preparing each nano/micro formulation. In more details, the independent variables included:

(i) **The water phase** made up of different quantities of D-fructose (D-fruc), polyethylene glycol 4000 (PEG), poly (vinyl alcohol) (PVA) 87% - 89% hydrolysed dispersed in deionised water (DW) represented as 0, 1 and 2 in Tables 1 and 2

(ii) **The oil phase** was composed of different quantities of ethyl cellulose - viscosity 22cP (Ethocel 22) (indicated as Eth 22 in Tables 1 and 2) dispersed in coconut oil (CO) represented as 3, 5 and 7 in Tables 1 and 2, and

(iii) **Homogenization velocity (HV)** refers to the speed of the externally applied mechanical blending force denoted in Tables 1 and 2

Combinations of the above-mentioned independent variables set at different levels were employed for the manufacturing of each nano/micro formulation so as to achieve the formation of a stable and effective set of formulations that can be suitably optimized for intended pharmaceutical application. The lower and upper limits selected for the independent variables were based on preliminary experiments. Tables 1 and 2 detail the 3-level of independent variables employed and the 3-factor, 3-centre points Box-Behnken experimental design template made up of 15 variant nano/micro formulations.

**Table** 1: Dependent and independent variables employed in the Box Behnken design

| Factor Levels | | | | |
|---|---|---|---|---|
| **Independent Variables** | | **-1** | **0** | **+1** |
| $X_1$ | Water phase (g) | 0 | 1 | 2 |
| $X_2$ | Oil phase (g) | 3 | 5 | 7 |
| $X_3$ | Homogenization velocity (rpm) | 4 | 6 | 8 |

| Dependent Variables | |
|---|---|
| $Y_1$ | Zeta Potential (mV) |
| $Y_2$ | Drug Entrapment Efficiency |
| $Y_3$ | Particle Size (nm) |

*NOTE: Water phase: 0 - deionised water = 15.0 g, fructose = 0.7 g, polyethylene glycol 4000 = 0.3 g, poly (vinyl alcohol) = 1.5 g; 1 - deionised water = 18.0g, fructose = 1.1 g, polyethylene glycol 4000 = 0.9 g, poly (vinyl alcohol) = 1.9 g; 2 - deionised water = 21 g, fructose = 1.5 g, polyethylene glycol 4000 = 1.5 g, poly (vinyl alcohol) = 2.3 g. Oil phase: 3 - ethyl cellulose = 0.5 g, coconut oil = 15.0 g, 5 - ethyl cellulose = 1.0 g, coconut oil = 10.0 g, 7 - ethyl cellulose = 1.5 g, coconut oil = 5.0 g. Homogenization velocity: 4 - 4000 rpm, 6 - 6000 rpm and 8 - 8000 rpm.*

**Table 2:** Box-Behnken design template for preparing the nano/micro formulations

| Formulation | DW (g) | D-fruc (g) | PEG (g) | PVA (g) | CO (g) | Eth 22 (g) | HV (rpm) |
|---|---|---|---|---|---|---|---|
| 1 | 18.0 | 1.1 | 0.9 | 1.9 | 5.0 | 1.5 | 4000.0 |
| 2 | 18.0 | 1.1 | 0.9 | 1.9 | 15.0 | 0.5 | 8000.0 |

(continued)

| Formulation | DW (g) | D-fruc (g) | PEG (g) | PVA (g) | CO (g) | Eth 22 (g) | HV (rpm) |
|---|---|---|---|---|---|---|---|
| 3 | 21.0 | 1.5 | 1.5 | 2.3 | 15.0 | 0.5 | 6000.0 |
| 4 | 15.0 | 0.7 | 0.3 | 1.5 | 10.0 | 1.0 | 4000.0 |
| 5* | 18.0 | 1.1 | 0.9 | 1.9 | 10.0 | 1.0 | 6000.0 |
| 6 | 15.0 | 0.7 | 0.3 | 1.5 | 15.0 | 0.5 | 6000.0 |
| 7* | 18.0 | 1.1 | 0.9 | 1.9 | 10.0 | 1.0 | 6000.0 |
| 8 | 18.0 | 1.1 | 0.9 | 1.9 | 15.0 | 0.5 | 4000.0 |
| 9* | 18.0 | 1.1 | 0.9 | 1.9 | 10.0 | 1.0 | 6000.0 |
| 10 | 15.0 | 0.7 | 0.3 | 1.5 | 5.0 | 1.5 | 6000.0 |
| 11 | 21.0 | 1.5 | 1.5 | 2.3 | 5.0 | 1.5 | 6000.0 |
| 12 | 21.0 | 1.5 | 1.5 | 2.3 | 10.0 | 1.0 | 4000.0 |
| 13 | 15.0 | 0.7 | 0.3 | 1.5 | 10.0 | 1.0 | 8000.0 |
| 14 | 21.0 | 1.5 | 1.5 | 2.3 | 10.0 | 1.0 | 8000.0 |
| 15 | 18.0 | 1.1 | 0.9 | 1.9 | 5.0 | 1.5 | 8000.0 |

**NOTE:** *Each formulation contained 1500 mg of isoniazid as model drug*
*Indicate the design centre points*

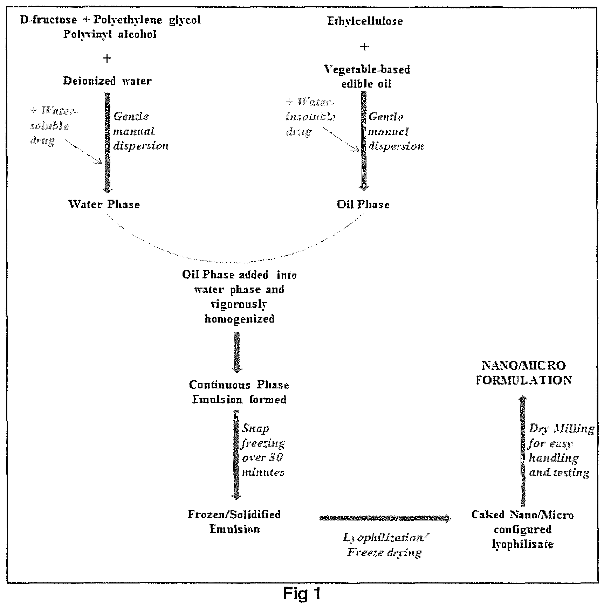

For each nano/micro formulation, the water phase containing D-fruc, PEG, PVA and the model drug at this stage, isoniazid, were dispersed in deionised water (DW) at different levels (Table 2) and the oil phase made up of varying quantities of Eth 22 dispersed in coconut oil (Table 2) were separately prepared under room conditions (23°C ± 2°C). Thereafter, the oil phase dispersion was gently added into the water phase and then immediately subjected to a rigorous, continuous blending in the presence of different magnitudes of an externally applied blending force of a mixer homogenizer for 5 minutes (Silverson Machines, Inc., Massachusetts, United States of America) to produce a monophasic emulsion. With D-fructose as a lyoprotectant/cryoprotectant, each formed emulsion formulation was then snap frozen by placing it in liquid nitrogen for about 20 minutes. Subsequently, frozen emulsion samples were then freeze dried/lyophilized (Benchtop Pro Freeze Dryer, VirTis, SP Scientific, New York, United States of America) at a temperature of -60 ± 2°C and pressure of 124 ± 2 mtorr (about 16.5 Pa(a)) for 96 hours to produce a solid lyophilisate/dried cake. For easy handling and testing, the produced lyophilisate was dry milled using a laboratory scale machine (Kinematica GMBH, Eschbach, Germany) and stored away in airtight and opaque containers for further testing. Figure 1 illustrates the direct dispersion emulsification method employed to produce the exemplified nano/micro formulations of the invention.

**Characterization of the 15 experimental design based nano/micro formulations**

*Determination of particle size, polydispersity index and zeta potential*

Measurement of particle size (diameter) and polydispersity index was based on the principle of dynamic light scattering using the Nano series Zetasizer equipped with the Zetasizer software, version 6.20 (Malvern Instruments Ltd, Malvern, UK). For all measurements, samples were re-dispersed in distilled water, appropriately diluted and sonicated (Table-type Supersonic Cleaner KQ118, Nanjing T-Bota Scietech Instruments and Equipment, Co, Ltd., Jiangsu, China) for 15 minutes at 37°C. All measurements were performed as three independent replicates with 15 readings per sample at a measurement angle of 173° and a temperature of 25°C.

The zeta potential, an indicator of particle surface charge which determines particle stability in dispersion was computed based on the Smoluchowski equation using the Nano series Zetasizer equipped with the Zetasizer software, version 6.20 (Malvern Instruments Ltd, Malvern, UK). Each sample was dispersed in an aqueous media and measurements were carried out in triplicate with 20 runs per each measurement cycle at 25°C.

*Drug loading efficiency*

To determine the amount of drug loaded within each nano/micro formulation variant, 10 mg of each sample was placed in 100 mL phosphate buffer (pH 7.4) and continuously stirred (Five-Position Hot Plate/Stirrer, Model 51450 series, Cole-Parmer, Illinois, United States of America) for 4 hours to ensure complete dissolution and release of entrapped drug molecules. The resultant solution was then appropriately diluted with distilled water and passed through a 0.45 μm pore size polypropylene membrane cameo syringe filter (Millipore Corporation, Massachusetts, United States of America). The actual drug content was analysed using Ultraviolet Spectrophotometry (PerkinElmer Lambda 35, UV/Vis Spectrometer, Perkin Elmer, Singapore) at a maximum wavelength of absorption for isoniazid, $\lambda_{max}$ = 262 nm. The percentage of isoniazid-loaded within each nano/micro formulation was mathematically computed with reference to the initial quantity that was added into each formulation. All tests were conducted as three replicate samples.

### Evaluation of formulation yield

Produced nano/micro formulations were weighed using a laboratory scale balance (Kern EG 620-3NM, Kern and Sohn, GmbH, Balingen, Germany). The percentage yield was calculated utilizing equation 1 below:

$$\%Yield = \frac{Total\ Weight\ of\ formulation\ (g)}{Total\ Weight\ of\ Component\ Compounds(g)} \times 100 \quad \dots\dots\dots\dots (1)$$

### In vitro dissolution studies

*In vitro* dissolution behaviour of the nano/micro formulations was evaluated employing the dissolution tester (Ewreka GmbH, DT 820 series, Heusenstamm, Germany). For each test, 500 mg of each nano/micro formulation was filled into an empty gelatine capsule shell (CapsCanada, Ontario, Canada), placed into the basket holder attached to the stirring shaft of the dissolution tester (United States Pharmacopeia Apparatus 1). The whole contrivance was then immersed into a dissolution jar containing 500 mL of pre-heated buffer solution (pH 1.2, pH 6.8 and pH 7.4 separately) at 37 $\pm$ 0.5°C rotating continuously at 100 rpm. 3 mL sample was collected at pre-determined time-points over 2 hours. Experiments were performed in triplicate under sink conditions. Collected samples were appropriately diluted, filtered using a 0.45 $\mu$m pore size polypropylene membrane cameo syringe filter (Millipore Corporation, Massachusetts, United States of America) and analysed spectrophotometrically (PerkinElmer Lambda 35, UV/Vis Spectrometer, Perkin Elmer, Singapore) at a $\lambda_{max}$ of 262 nm to determine the amount of isoniazid contained in every withdrawn sample. Percentage drug release was computed relative to the total amount of isoniazid present within the dissolution medium.

### Systematic statistical optimization based on the Box-Behnken design template

Experimental outputs from the Box-Behnken quadratic design template relating independent with dependent variables were subjected to strict settings for predicting the optimal formulation. A synchronized optimization approach was applied utilizing the response surface optimizer (Minitab software, Version 16, Pennsylvania, United States of America). In this regard, limits were established in order to generate independent variables that will concurrently influence the response parameters to generate the desired statistically relevant optimal levels. Consequently, a target was set for the drug entrapment efficiency, zeta potential while particle size was minimized (Table 3). A composite desirability value of 0.99 which indicated the robustness and accuracy of the optimizer was obtained. An estimation of the statistical significance and dependability of the model was evaluated using the one-way analysis of variance (ANOVA) with p-values set at 95% confidence level ($p \leq 0.05$) and correlation coefficient selected as key indicators ($R^2 > 0.80$).

**Table 3:** Numerical optimization parameters set for the selected responses

| Parameters | Optimization Goal | Lower | Target | Upper | Desirability |
|---|---|---|---|---|---|
| Zeta Potential (mV) | Target | 38.00 | 40.00 | 42.00 | 0.99 |
| Drug loading efficiency (%) | Target | 92.00 | 96.00 | 98.00 | 1.00 |
| Particle size (nm) | Minimize | 400.00 | 500.00 | 700.00 | 1.00 |

### Preparation and physicochemical characterization of the optimized formulation

### Optimized formula

Based on the statistical optimization process described, an optimized nano/micro formulation was developed. The formula of the optimized formulation is highlighted in Table 4. The optimized nano/micro formulation was prepared following the direct dispersion emulsification technique illustrated in Figure 1.

Table 4: Composition of the statistically optimized formulation

| Optimized Formula Components | Quantities |
|---|---|
| Deionised water | 18.0 g |
| D-Fructose | 1.1 g |
| Polyethylene glycol | 0.9 g |
| Polyvinyl alcohol | 1.9 g |
| Coconut oil | 10.0 g |
| Ethyl cellulose | 1.0 g |
| Model drug (isoniazid) | 1.5 g |
| Homogenization velocity | 4000.0 rpm |

### *Physicochemical characterization of the optimized nano/micro formulation*

Particle size, polydispersity index and zeta potential were determined based on the method already described hereinbefore. The evaluations of drug loading efficiency, formulation yield and in *vitro* dissolution were also performed in accordance with the methods outlined in hereinbefore. All analyses were conducted three times. With drug release, the duration of dissolution was increased to 4 hours.

The morphology of the optimized formulation was visualized using Transmission Electron Microscopy (TEM). About 0.5 mg of test sample was dispersed in ethanol and spotted on top of a carbon coated copper grid. The ethanol was allowed to evaporate under atmospheric conditions before the samples were loaded into the TEM machine viewing stage (JEOL JEM-2100 LaB6 200 kV Transmission Electron Microscope, JEOL, Massachusetts, United States of America). Sample viewing under the TEM machine was facilitated by the Gatan Digital Micrograph Software (Gatan Inc. California, United States of America).

A qualitative powder x-ray diffraction (XRD) study was performed on the optimized formulation under room temperature conditions employing an X-ray diffractometer equipped with the X'Pert PRO data collector software (PANalytical Inc. Massachusetts, United States of America). Approximately 2 - 4 mg of the sample powder were loaded on standard sample holders and tested. The samples were continuously scanned between a range of 5.0" and 90.5° with a scan step size of 0.03.

Fourier Transform Infra-red (FTIR) spectra of the optimized formulation were generated on a Perkin Elmer Spectrum 100 Series FTIR Spectrophotometer coupled with the Spectrum V 6.2.0 software (Beaconsfield, UK). The sample holder situated on the test stage of the FTIR machine was cleaned with ethanol and allowed to dry properly. Thereafter, 5 mg of the test sample was placed on the cleaned stage for structural analysis. Blank background readings were taken prior to sample analysis which was carried out with wavenumbers ranging from 4000-650 cm$^{-1}$, scan time = 32 scans and resolution of 4 cm$^{-1}$. Recorded outputs were computed as an average of three repeated scans.

### Evaluating the effect of loading a hydrophobic drug, rifampicin, onto the optimized nano/micro formulation

As set out hereinbefore, isoniazid, a hydrophilic molecule, was explored as the model drug. In order to evaluate the versatility of the direct dispersion emulsification technique to enable the formulation of hydrophilic or hydrophobic bioactive molecules, rifampicin, a hydrophobic molecule was incorporated into the optimized formulation and tested accordingly. Characteristic tests performed included drug loading efficiency, yield, particle analysis (size, polydispersity index and zeta potential), morphology and drug release. These tests were conducted based on the methods described hereinbefore. Outcomes of these measurements were compared with those generated for the isoniazid-containing formulation. For drug release and drug loading efficiency analyses for rifampicin, the wavelength of absorption, $\lambda_{max}$ = 338 nm, which is characteristic for this molecule was employed. The quantity of rifampicin obtainable at this maximum wavelength was mathematically computed. Furthermore, drug release analysis was performed over a duration of 6 hours to ensure that the rifampicin molecules are completed released from the within the formulation matrix.

### Preparation of the optimized formulation using a variety of vegetable-based edible oils other than coconut oil

Furthermore, the versatility of the direct dispersion emulsification technique with regards to varying the oilphase which is based on the nature of the vegetable-based oil employed in the manufacture of the stable nano/micro formulation was also explored. The formulas evaluated were based on the basic composition of the optimized formulation (Table 4) and are presented in Table 5 below. The produced formulations were characterized and compared with the earlier-described coconut oil-based formulation by measuring particle size, polydispersity index, zeta potential, formulation yield and drug loading efficiency in triplicate.

**Table 5:** The statistically optimized formulation prepared with a variety of vegetable based oils to assess the versatility of the direct dispersion emulsification method

| Types of Vegetable Oil | Formulation Abbreviation |
| --- | --- |
| Coconut oil | Opt-CO |
| Corn oil | Opt-CRO |
| Olive oil | Opt-OO |
| Peanut oil | Opt-PO |
| Soybean oil | Opt-SO |

*Other formulation components are exactly the same as reported in Table 4 above. For each formulation, 10 g of the respective vegetable based oil is used. The model drug employed in assessing the performance of these formulations was isoniazid.*

**Stability Testing**

*Evaluation of stability under different environmental conditions*

Stability testing was carried out on both the isoniazid-loaded and rifampicin-loaded optimized formulations. The different environmental conditions evaluated were based on three different storage settings:

i) **In a stability tester:** The samples (500.0 mg $\pm$ 2 mg) were placed in an enclosed glass holder and into a stability tester (Labcon PSIE RH 40 Chamber Standard Incubator, Laboratory Marketing Services, Maraisburg, South Africa) fixed at settings of 30°C $\pm$ 2°C and a relative humidity of 65% $\pm$ 3% adapted from the World Health Organization stability testing scheme for pharmaceutical products containing well established drug substances.

ii) **Under room conditions:** Samples (500.0 mg $\pm$ 2 mg) were stored in airtight, opaque glass vials within room conditions (25°C $\pm$ 5°C and relative humidity 55% $\pm$ 5%) for 4 months.

iii) **In a refrigerator:** Samples (500.0 mg $\pm$ 2 mg) were placed in airtight, opaque glass vials and put into a refrigerator (Sanya Labcool Pharmaceutical Refrigerator, MPR-720R, Sanyo Electrical Biomedical Co. Ltd, California, United States of America) at a temperature of 4°C $\pm$ 1°C.

Indicators of formulation stability under the different set test conditions were particle size, polydispersity index, zeta potential, drug content (DC) and physically examined sample colour evaluated at 0, 1 and 4 months intervals as three replicate samples.

*Assessment of hydrostability in aqueous suspension environment*

Hydrostability, which is indicative of the stability of the isoniazid or rifampicin formulation when exposed to aqueous conditions (e.g. during re-constitution or re-suspension) was also evaluated. Powdered samples (500 mg) were contained in airtight, opaque containers and dispersed in sterilized deionised water (50 mL). Each hydrated test sample was prepared in triplicate and samples were placed under room conditions (23"C $\pm$ 2°C) as well as concurrently in the refrigerator (4°C $\pm$ 2°C). The test was carried out for 11 days and samples (2 mL) were collected at predetermined time intervals (0, 1, 5 and 11 days) to assess drug content in triplicate. Prior to the sample collection and measurement stages, samples were subjected to gentle manual shaking to ensure uniform re-dispersion.

**Experimental results**

**Quantification of the physical properties of the 15 experimental design nano/micro formulations**

The nano/micro formulations produced based on the Box-Behnken experimental design template highlighted in Table 2 generated response parameters which are indicative of their varying measured physical properties. The dependent variable (responses) studied included particle size or diameter (PS), polydispersity index (PDI), zeta potential (ZP), drug loading efficiency (DLE), formulation yield ($F_{yield}$) and cumulative drug release over 2 hours in buffer media with variable pH of 1.2 (CDR$_{2hrs}$1.2), pH 6.8 (CDR$_{2hrs}$6.8) and pH 7.4 (CDR$_{2hrs}$7.4) and the values are outlined in Table 6.

The 15 nano/micro experimental design formulations appeared as cream-white free flowing, powdery solids. Particle sizes or diameters of the experimental design formulations spanned over the nano-range of 400.10 nm $\pm$ 8.767 nm and the micro-range of 1624.00 nm $\pm$ 19.42 nm with a polydispersity index that was between 0.37 $\pm$ 0.011 and 0.91 $\pm$ 0.117 depending on the composition of the respective nano/micro formulation (Table 6). The zeta potential values outlined in Table 6 showed that the particulate components of the nano/micro formulations displayed an overall negatively charged surface characteristics with good stability potential for dispersion as suspensions as all the formulations excluding F6 (-23.50 mV $\pm$ 1.05 mV) had zeta potential values above 30.00 mV. Research has shown that particulate formulations with zeta potential values greater than ($\pm$) 30.00 mV are stable in suspension. Drug loading within the particulate content of each nano/micro formulation can be described as quite efficient with values that ranged between 70.04% $\pm$ 5.22% and 107.63% $\pm$ 3.15% (Table 6) and overall average of 88.84%. The direct dispersion emulsion process can be described as high yielding with regards to the final quantity of the nano/micro formulations produced with a minimum and maximum yield of 87.43% $\pm$ 0.01% and 95.25% $\pm$ 0.11% respectively (Table 6). The formulations displayed diverse drug release behaviours in the different phosphate buffered media (pH 1.2, 6.8, 7.4) that were studied. Drug release behaviour was characterized with a "burst release" for all formulations followed by consistent release over the 2 hour test duration. Drug release capacity under each release media condition varied from one formulation to the other (Table 6). Drug release mimicked a zero order pattern with values of cumulative drug release at 2 hours ranging from 43.97% $\pm$ 2.10% 84.06% $\pm$ 3.33% (pH 1.2 media), 43.54% $\pm$ 0.57% - 83.77% $\pm$ 1.01% (pH 6.8 media) and 33.07% $\pm$ 1.47% - 84.49% $\pm$ 2.84% (pH 7.4 media).

**Physicochemical Characterization of the isoniazid-loaded optimized nano/micro formulation**

The optimized nano/micro formulation was prepared in accordance with the formula systematically derived through the statistical optimization procedure (Table 4). The optimized formulation had an average particle size of 428.14 nm $\pm$ 25.54 nm, zeta potential of - 38.68 mV $\pm$ 3.21 mV, polydispersity index of 0.38 $\pm$ 0.04, drug loading efficiency of 97.13% $\pm$ 1.05% and process yield of 98.02% $\pm$ 0.01%. The zeta potential, drug loading efficiency and particle size were initially selected as pointers for the statistical optimization of the nano/micro formulation (Table 3). The obtained experimental values were closely related to the statistically predicted values (see Table 7 below). This outcome revealed that the high performance quadratic experimental design template selected for the formulation optimization was not only suitable for its intended application but was also accurate, stable and robust.

Table 6: Numerical values of the response parameters generated for the 15 experimental design Nano/micro formulations

| Nano/Micro Formulations | PS (nm) [a] | PDI [b] | ZP (mV) [c] | DLE (%) [d] | F$_{yield}$ (%w/w) [e] | CDR$_{2hrs}$1.2 (%)[f] | CDR$_{2hrs}$6.8 (%) [g] | CDR$_{2hrs}$7.4 (%) [h] |
|---|---|---|---|---|---|---|---|---|
| 1 | 525.10 | 0.46 | -38.10 | 83.67 | 94.21 | 84.06 | 79.55 | 77.42 |
| 2 | 439.40 | 0.38 | -34.20 | 101.30 | 92.88 | 45.26 | 43.54 | 33.07 |
| 3 | 901.60 | 0.69 | -32.90 | 97.92 | 92.92 | 79.46 | 80.87 | 62.87 |
| 4 | 400.10 | 0.37 | -35.20 | 85.04 | 89.85 | 75.94 | 83.77 | 63.70 |
| 5 | 498.24 | 0.43 | -42.41 | 92.05 | 93.90 | 70.92 | 54.08 | 47.34 |
| 6 | 1022.20 | 0.79 | -23.50 | 91.89 | 88.34 | 78.49 | 80.85 | 62.56 |
| 7 | 499.04 | 0.44 | -40.61 | 93.45 | 94.82 | 71.33 | 54.96 | 49.38 |
| 8 | 492.40 | 0.43 | -40.71 | 81.71 | 90.15 | 57.63 | 71.89 | 48.77 |
| 9 | 497.98 | 0.44 | -39.96 | 93.49 | 94.85 | 71.33 | 55.91 | 49.80 |
| 10 | 484.01 | 0.53 | -38.90 | 70.04 | 95.25 | 43.97 | 68.32 | 67.44 |
| 11 | 578.06 | 0.45 | -40.80 | 80.42 | 93.33 | 62.60 | 61.31 | 84.49 |
| 12 | 1624.00 | 0.38 | -41.12 | 94.12 | 87.43 | 67.88 | 60.18 | 49.36 |
| 13 | 1066.05 | 0.91 | -46.70 | 79.97 | 92.46 | 66.94 | 68.10 | 65.13 |
| 14 | 949.90 | 0.75 | -45.51 | 94.42 | 94.45 | 69.72 | 56.78 | 48.25 |

(continued)

| Nano/Micro Formulations | PS (nm) [a] | PDI [b] | ZP (mV) [c] | DLE (%) [d] | F_yield (%w/w) [e] | CDR_{2hrs}1.2 (%) [f] | CDR_{2hrs}6.8 (%) [g] | CDR_{2hrs}7.4 (%) [h] |
|---|---|---|---|---|---|---|---|---|
| 15 | 946.11 | 0.74 | -46.30 | 68.89 | 91.55 | 53.93 | 69.19 | 60.61 |

[a] Particle Size (Standard Deviation $\leq$ 223.50 nm in all cases), [b] Polydispersity Index (Standard Deviation $\leq$ 0.16 in all cases), [c] Zeta Potential (Standard Deviation $\leq$ 10.60 mV in all cases), [d] Drug Loading Efficiency (Standard Deviation $\leq$ 16.88% in all cases), [e] Formulation Yield (Standard Deviation $\leq$ 0.54% in all cases), [f] Cumulative Drug Release (pH 1.2) (Standard Deviation $\leq$ 6.83% in all cases), [g] Cumulative Drug Release (pH 6.8) (Standard Deviation $\leq$ 9.72% in all cases), [h] Cumulative Drug Release (pH 7.4) (Standard Deviation $\leq$ 7.14% in all cases) (N = 3 in all cases).

**Table 7:** Comparison of predicted versus experimental values of the optimization parameters

| Optimization Parameters | Predicted Values | Experimental values |
|---|---|---|
| Zeta Potential (mV) | -40.00 | -38.68 |
| Drug loading efficiency (%) | 96.00 | 97.13 |
| Particle size (nm) | 500.00 | 428.14 |

In *vitro* isoniazid release from the optimized nano/micro formulation was characterized with an initial burst from 5 minutes (10.51%) which increased at a consistent rate up until 40 minutes, followed a slower significant increase in percentage release up to 60 minutes after which a plateau was reached where drug release remained more or less consistent up to the 240 minutes time point when about a 100% release was achieved. To further analyse the *in vitro* release profile, a variety of kinetic models were utilized for the model fitting process using free open source software (KinetDS, version 3.0). The selection of the choice model which fitted the release data the best was based on the correlation coefficient ($R^2$) with values closest to 1. On this basis, the *in vitro* drug release kinetics of the isoniazid-loaded optimized nano/micro formulation was best expressed by the Michaelis-Menten ($R^2$ = 1.00) and Korsmeyer Peppas ($R^2$ = 0.99) equation because both fits displayed the best linearity. These mathematical models indicate that drug release is mono-dimensional from the polymer-based platform. Besides, the diffusion exponent (n) displays values greater than 0.89 with the Michaelis-Menten model, n = 1.00 and the Korsmeyer Peppas model, n = 1.01. The obtained n values signify that the release trend follows a super case II transport mechanism. Consequently, it can be inferred that isoniazid release from the optimized nano/micro formulation is controlled by stress induced relaxation and state transition within the polymeric chain resulting in swelling followed by erosion of polymeric chain and irregular diffusion of drug molecules.

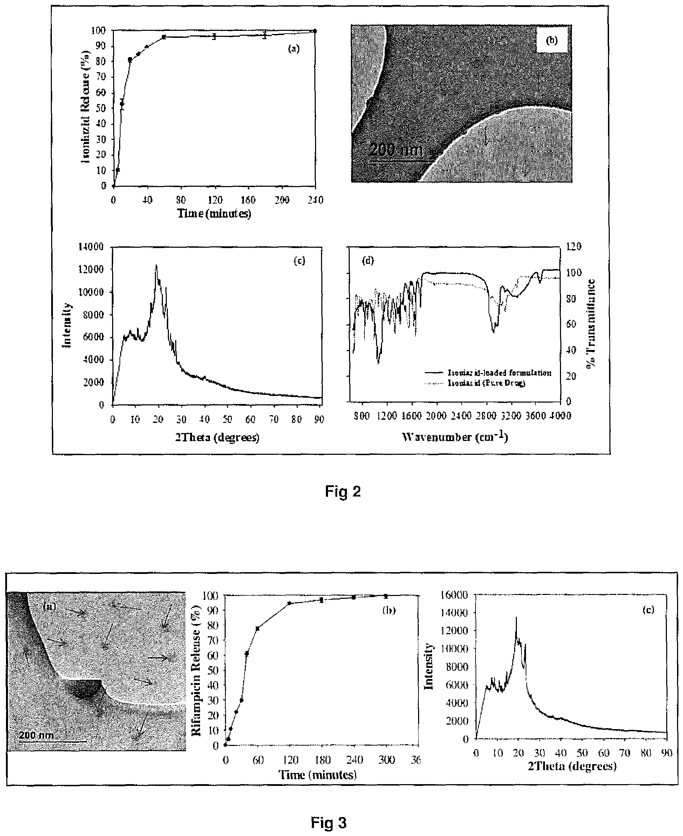

The TEM image of the isoniazid-loaded optimized nano/micro formulation is shown in Figure 2 (b). The particulate composition of the nano/micro formulation can be described as spherical in geometry (highlighted by the red arrows in Figure 2 (b)). The powder XRD diffractogram of the formulation is shown in Figure 2 (c). The characteristic broad peaks observed from the generated XRD diffractogram showed that the isoniazid-loaded nano/micro formulation can be referred to as semi-crystalline in nature. The observed diffraction pattern (Figure 2 (c)) showing a number of different blunt peaks further indicate that the formation of the nano/micro formulation is not chemical in nature but due to a physical interaction between the individual component compounds. Furthermore, the nature of the produced diffractogram peaks confirms the presence of polymeric components within the formulation. The semi-crystalline nature of this formulation can explain why its dissolution rate is relatively quick based on its drug release behaviour which is characterised by the initially elicited burst effect followed by almost a 100% release in about 2 hours (Figure 2(a)).

The peaks depicting characteristic vibrational frequencies of isoniazid in its pure state and loaded in the formulation were compared. For pure isoniazid, bond vibrational peaks recorded included C-C-C-C and C-N-C-C torsion (653 cm$^{-1}$), $NH_2$ rock (677 cm$^{-1}$) C-C-C and C-C-H out of plane bending (746 cm$^{-1}$), C-C-H out of plane bending (850 cm$^{-1}$; 1022 cm$^{-1}$), C-N-C and C-C-C in plane bending (1063 cm$^{-1}$), C-C-H in plane bending (1210 cm$^{-1}$), O=C-N and C-C-H in plane bending (1330 cm$^{-1}$), C-N-H in plane bending (1411 cm$^{-1}$), C-C stretching (1477 cm$^{-1}$), C=O stretching (1558 cm$^{-1}$), $NH_2$ scissoring (1632 cm$^{-1}$), aromatic C-H stretching (3052 cm$^{-1}$) and N-H stretch (3307 cm$^{-1}$). The vibrational peaks documented for isoniazid in the nano/micro formulation were C-C-C-C and C-N-C-C torsion (665 cm$^{-1}$), $NH_2$ rock (677 cm$^{-1}$) C-C-C and C-C-H out of plane bending (757 cm$^{-1}$), C-C-H out of plane bending (850 cm$^{-1}$; 1023 cm$^{-1}$), C-N-C and C-C-C in plane bending (1062 cm$^{-1}$), C-C-H in plane bending (1212 cm$^{-1}$), O=C-N and C-C-H in plane bending (1333 cm$^{-1}$), C-N-H in plane bending (1408 cm$^{-1}$), C-C stretching (1471 cm$^{-1}$), C=O stretching (1552 cm$^{-1}$), $NH_2$ scissoring (1635 cm$^{-1}$), aromatic C-H stretching (3054 cm$^{-1}$) and N-H stretch (3308 cm$^{-1}$). Comparing the two sets of vibrational frequency readings for isoniazid *in* the pure and formulation states, a closely related trend in the magnitudes of the vibrational frequencies is notable. These similarities indicate that the nano/micro formulation is a physical mixture of active drug plus excipients and not a chemical, irreversible or destructive interaction among the component compounds.

**Characterization of the rifampicin-loaded optimized nano/micro formulation**

The rifampicin-loaded formulation had a drug loading efficiency of 98.83 $\pm$ 1.23%, a process yield of 98.87 $\pm$ 0.02% with an average particle size of 429.37 $\pm$ 28.65 nm, zeta potential of -36.10 $\pm$ 2.89 mV and polydispersity index of 0.37 $\pm$ 0.03. Particulate geometry of the rifampicin-loaded formulation can be described as spherical as illustrated with the arrows in Figure 3 (a) from the TEM micrograph.

*In vitro* drug release (Figure 3 (b)) followed a similar pattern with that of the isoniazid-loaded formulation but with an extended duration. Drug release behaviour of the rifampicin-loaded formulation was also characterized by a burst release which was initiated at 5 minutes (3.93%) and increased steadily until 120 minutes (94.46%) after which drug release was more or less consistent up to 360 minutes when about a 100% release was achieved. In a nutshell, the rifampicin release pattern from the optimized nano/micro formulation followed a similar trend when compared with the isoniazid-loaded formulation. However, rifampicin release rate was slower than that of isoniazid and this is attributable to difference in the aqueous solubility of both model drugs. Additionally, the release profile of the rifampicin-loaded formulation was subjected to model fitting procedures employing the free open source software (KinetDS, version 3.0). The best fit was based on the value of the correlation coefficient ($R^2$) with values closest to 1. With reference to this, the *in vitro* release mechanism of this formulation was best explained by the Michaelis-Menten ($R^2$ = 1.00) and Korsmeyer Peppas ($R^2$ = 0.97) based on the $R^2$ values. This indicates that drug release from the formulation is unidimensional. Also the n (diffusion exponent) values > 0.89 (Michaelis-Menten model, n = 0.99 and the Korsmeyer Peppas model, n = 1.20) implies that the drug release mechanisms of the rifampicin-loaded formulation is also regulated by stress induced relaxation and polymeric chain transition which leads to matrix swelling, erosion and diffusion modulation (super case II transport). Overall, the optimized nano/micro formulation is a robust and flexible carrier system that can effectively regulate the release of either hydrophilic or hydrophobic drugs/bioactive moieties.

The nature of the powder XRD diffractogram (Figure 3(c)) was majorly characterised by blunt, not well defined peaks (amorphous domains) in terms of sharpness and a few distinct peaks (crystalline domains) showing that the formulation is semi-crystalline and contains multiple compounds. This indicates that no irreversible chemical transitions occurred during the production of the rifampicin-loaded nano/micro formulation. This also confirms the nature of the release profile which is characterised as a relatively quick drug release rate.

**Testing of optimized nano/micro formulation prepared with alternate vegetable-based oil phase other than coconut oil**

The produced drug nano/micro formulation variants based on alternate vegetable oils (corn, olive, peanut and soybean oils) as oil phases had a high process yield (95.56% - 99.81%), drug loading capacity (93.83%-98.77%), nano-range particulate dimensions of 277.96 nm - 718.58 nm, polydispersity index ranging between 0.32 - 0.47 and zeta potential of 29.85 mV - 34.84 mV (Table 8). These values are related to the values obtained for the optimized formulation with oil phase solely made up of coconut oil.

**Table 8:** Physicochemical characteristics of the optimized nano/micro formulation prepared using alternative vegetable-based edible oil

| Oil Phase Type | Physicochemical Properties | | | | |
|---|---|---|---|---|---|
| | Yield (%) | DLC (%) | PS (nm) | PDI | ZP(mV) |
| Coconut oil* | 98.02 $\pm$ 0.01 | 97.13 $\pm$ 1.05 | 428.14 $\pm$ 25.54 | 0.38 $\pm$ 0.04 | -38.68 $\pm$ 3.21 |
| Corn oil | 99.81 $\pm$ 0.03 | 93.83 $\pm$ 1.96 | 364.90 $\pm$ 32.11 | 0.32 $\pm$ 0.01 | -29.85 $\pm$ 1.15 |
| Olive oil | 95.56 $\pm$ 1.41 | 98.37 $\pm$ 1.54 | 419.98 $\pm$ 10.09 | 0.39 $\pm$ 0.02 | -34.84 $\pm$ 0.79 |
| Peanut oil | 99.05 $\pm$ 0.02 | 98.64 $\pm$ 1.15 | 718.58 $\pm$ 30.55 | 0.47 $\pm$ 0.07 | -30.11 $\pm$ 5.81 |
| Soybean oil | 96.81 $\pm$ 1.71 | 98.77 $\pm$ 0.96 | 277.96 $\pm$ 23.74 | 0.33 $\pm$ 0.01 | -30.67 $\pm$ 1.91 |
| *This represents the original/lead optimized nano/micro formulation | | | | | |

**Formulation Stability Evaluation**

*Varying environmental condition stability testing*

Outcomes of the performed stability test measured by the values of the stability indicators (polydispersity index, zeta potential, particle size, drug content and colour change) are presented in Tables 9 (a), 9 (b) and 9 (c). After 4 months of storage of the drug loaded optimized formulations, slight, statistically insignificant ($p > 0.05$) changes in the values of the stability indicators were noticed for all three testing conditions (stability tester - 30°C $\pm$ 2°C and 65% $\pm$ 3%, room - 25°C $\pm$ 5°C and 55% $\pm$ 5% and refrigerator - 4°C $\pm$ 1°C). However a slight colour change from pure white to cream-white was observed for the isoniazid-loaded formulation stored under room and stability tester conditions (Tables 9 (a) and (b)). Nevertheless, both model drugs were well preserved within the formulation under all storage conditions. Considering all the stability indicators measured under all the respective storage condition, storage of this formulation under refrigeration conditions (4°C $\pm$ 1°C) (Table 9 (c)) appears to be the most suitable storage conditions, as no discoloration was observed for all the formulations and changes in physical properties were slight and statistically insignificant ($p > 0.05$).

**Table 9 (a):** Values of stability indicators at the different time-points for stability experiments conducted within the stability tester environment

| Nano/Micro Formulation | Test Time Points (months) | Stability indicators | | | | |
|---|---|---|---|---|---|---|
| | | PDI[a] | ZP[b] (mV) | PS[c] (nm) | DC[d] (%) | Colour changes |
| | 0 | 0.38 | -38.68 | 428.14 | 97.13 | None |
| | 1 | 0.41 | -35.59 | 429.50 | 97.07 | None |
| **Isoniazid** | 4 | 0.39 | -33.60 | 403.55 | 96.76 | Slight |
| **Rifampicin** | 0 | 0.37 | -36.10 | 429.37 | 98.83 | None |
| | 1 | 0.36 | -35.80 | 402.20 | 98.32 | None |
| | 4 | 0.35 | -35.10 | 410.71 | 98.70 | None |

[a] Particle Size (Standard Deviation $\leq$ 14.6.1 nm in all cases), [b] Polydispersity Index (Standard Deviation $\leq$ 0.02 in all cases), [c] Zeta Potential (Standard Deviation $\leq$ 3.21 mV in all cases), [d] Drug Content (Standard Deviation $\leq$ 1.23% in all cases) ($N = 3$ in all cases).

**Table 9 (b):** Values of stability indicators at the different time-points for stability experiments conducted under room conditions

| Nano/Micro Formulation | Test Time Points (months) | Stability indicators | | | | |
|---|---|---|---|---|---|---|
| | | PDI[a] | ZP[b] (mV) | PS[c] (nm) | DC[d] (%) | Colour changes |
| | 0 | 0.38 | -38.68 | 428.14 | 97.13 | None |
| | 1 | 0.44 | -39.7 | 439.9 | 96.95 | None |
| **Isoniazid** | 4 | 0.46 | -37.1 | 419.78 | 97.05 | Slight |
| **Rifampicin** | 0 | 0.37 | -36.10 | 429.37 | 98.83 | None |
| | 1 | 0.39 | -34.30 | 409.40 | 98.04 | None |
| | 4 | 0.36 | -33.96 | 403.70 | 97.99 | None |

[a] Particle Size (Standard Deviation $\leq$ 10.11 nm in all cases), [b] Polydispersity Index (Standard Deviation $\leq$ 0.04 in all cases), [c] Zeta Potential (Standard Deviation $\leq$ 1.85 mV in all cases), [d] Drug Content (Standard Deviation $\leq$ 2.21% in all cases) ($N = 3$ in all cases).

**Table 9 (c):** Values of stability indicators at the different time-points for stability experiments conducted in the refrigerator environment

| Nano/Micro Formulation | Test Time Points (months) | Stability indicators | | | | |
|---|---|---|---|---|---|---|
| | | PDI[a] | ZP[b] (mV) | PS[c] (nm) | DC[d] (%) | Colour changes |
| | 0 | 0.38 | -38.68 | 428.14 | 97.13 | None |
| | 1 | 0.38 | -36.65 | 426.10 | 96.95 | None |
| Isoniazid | 4 | 0.35 | -35.91 | 431.30 | 97.02 | None |
| Rifampicin | 0 | 0.37 | -36.10 | 429.37 | 98.83 | None |
| | 1 | 0.37 | -34.21 | 418.85 | 98.88 | None |
| | 4 | 0.36 | -34.98 | 411.99 | 99.01 | None |

*[a] Particle Size (Standard Deviation $\leq$ 12.62 nm in all cases), [b] Polydispersity Index (Standard Deviation $\leq$ 0.01 in all cases), [c] Zeta Potential (Standard Deviation $\leq$ 1.43 mV in all cases), [d] Drug Content (Standard Deviation $\leq$ 1.48% in all cases) (N = 3 in all cases).*

### Hydrostability evaluation

The isoniazid and rifampicin loaded formulations were stable in the aqueous medium over the specified test period (11 days) and test condition (ambient and refrigerator) based on the values of the measured stability indicators outlined in Table 10. The drug content did not change significantly showing that the encapsulated model drugs were stable within the hydrated nano/micro matrix. Furthermore, this outcome reveals the usefulness of this nano/micro formulation in compounding pharmaceutical suspensions for re-constitution purposes.

**Table 10:** Values of stability indicators measured at different time-points visualizing the impact of an aqueous environment on the stability of the optimized formulation stored under ambient or refrigerated conditions

| Test Conditions | Nano/Micro Formulation | Test Time Point (days) | Hydrostability Indicators | | | | |
|---|---|---|---|---|---|---|---|
| | | | DC[a] (%) | ZP (mV) | PS (nm) | PDI | Discoloration |
| | | 0 | 97.13 $\pm$ 1.23 | -38.68 $\pm$ 1.43 | 428.14 $\pm$ 12.62 | 0.38 $\pm$ 0.01 | None |
| | | 1 | 97.18 $\pm$ 0.78 | -38.01 $\pm$ 0.88 | 433.45 $\pm$ 8.42 | 0.36 $\pm$ 0.06 | None |
| | Isoniazid | 5 | 96.66 $\pm$ 0.56 | -36.55 $\pm$ 3.25 | 456.10 $\pm$ 6.53 | 0.37 $\pm$ 0.04 | None |
| ROOM/AMBIENT | | 11 | 96.98 $\pm$ 1.99 | -35.91 $\pm$ 0.58 | 481.80 $\pm$ 1.32 | 0.39 $\pm$ 0.01 | None |
| | | 0 | 98.83 $\pm$ 2.05 | -36.10 $\pm$ 1.26 | 429.37 $\pm$ 12.11 | 0.37 $\pm$ 0.02 | None |
| | | 1 | 97.16 $\pm$ 1.88 | -37.22 $\pm$ 2.77 | 396.09 $\pm$ 8.11 | 0.38 $\pm$ 0.04 | None |

(continued)

| Test Conditions | Nano/Micro Formulation | Test Time Point (days) | Hydrostability Indicators | | | | |
|---|---|---|---|---|---|---|---|
| | | | DC[a] (%) | ZP (mV) | PS (nm) | PDI | Discoloration |
| | Rifampicin | 5 | 96.99 ± 2.13 | -36.85 ± 0.99 | 416.14 ± 4.76 | 0.36 ± 0.01 | None |
| | | 11 | 97.94 ± 1.87 | -37.09 ± 2.14 | 426.31 ± 5.63 | 0.37 ± 0.03 | None |
| | | 0 | 97.13 ± 1.23 | -37.02 ± 1.76 | 428.14 ± 12.62 | 0.38 ± 0.01 | None |
| | | 1 | 95.29 ± 1.02 | -37.91 ± 0.97 | 423.45 ± 4.01 | 0.38 ± 0.02 | None |
| | Isoniazid | 5 | 94.81 ±0.69 | -37.79 ± 3.56 | 448.96 ± 4.27 | 0.37 ± 0.01 | None |
| REFRIGERATOR | | 11 | 94.19 ± 2.01 | -37.53 ± 1.22 | 472.20 ± 4.44 | 0.37 ± 0.01 | None |
| | | 0 | 98.83 ± 2.05 | -36.10 ± 1.26 | 429.37 ± 12.11 | 0.37 ± 0.02 | None |
| | | 1 | 98.99 ±1.02 | -37.55 ± 2.26 | 398.92 ± 7.35 | 0.38 ± 0.05 | None |
| | Rifampicin | 5 | 98.54 ± 2.55 | -36.89 ± 3.01 | 408.09 ± 4.52 | 0.37 ± 0.01 | None |
| | | 11 | 98.97 ± 0.89 | -37.02 ± 1.76 | 426.30 ± 1.23 | 0.38 ± 0.03 | None |

The method of the invention advantageously can provide nano/micro-sized environmentally stable pharmaceutical formulations in an oil-polymer carrier system, combining an oleo-polymeric technique with lyophilisation and optionally a dry milling process. The method of the invention, as illustrated, uses an oleo-polymeric technique which is conservative, simple and convenient, involving a direct dispersion emulsification approach which makes use of less stringent room conditions, in particular room temperature for mixing/emulsification, and use biologically compatible solvent systems such as water and edible vegetable oils that are free of toxic organic solvents and biocompatible and biodegradable United States Food and Drug Administration (USFDA), Generally Recognized as Safe (GRAS) polymeric and non-polymeric additives. The pharmaceutical formulations are formed by physical, non-destructive interactions between pharmaceutical active and polymeric and non-polymeric excipients. The ingredients are agitated in the presence of a carefully adjusted externally applied blending force. Advantageously, the method of the invention, as illustrated, produces stable nano/micro configured formulations with attractive qualities, which are useful for drug delivery applications, such as very high loading efficiency and product yield, controlled release and good transmembrane permeation characteristics, flexible characteristics in the sense that it can load either hydrophobic or hydrophilic drug molecules as well as accommodate the use of different encapsulating materials such as the edible vegetable oils and polymeric and non-polymeric additives. The formulations produced by the method of the invention, as illustrated, undergo stress induced polymeric chain relaxation/transition followed by matrix swelling, erosion and diffusion to release drug molecules. The pharmaceutical formulations prepared by the method of the invention, as illustrated, have the capability of regulating the influx of body fluids into their matrices and therefore to release the encapsulated pharmaceutical active at different administrations sites or through different administration routes, such as via the oral route or via the transmucosal route.

Generally, conventional methods of micro/nano encapsulation in contrast are cumbersome, labour intensive, requires strict conditions relating to temperature and pressure and the like, are performed in the presence of toxic solvents, are difficult to scale-up, are expert-dependent and low-yielding, have a low encapsulation efficiency and are non-flexible in terms of loading hydrophilic and hydrophobic drugs. Overall, the method of the invention, as illustrated, can provide pharmaceutical formulations that can be applied orally (e.g. in the form of capsules or suspensions) and/or are oromucosal dispersible (e.g. in the form of films, gels or wafers).

**Claims**

1. A method for encapsulating a pharmaceutical active, the method including
   dispersing a hydrophilic polymeric matrix former into a water phase and a hydrophobic polymeric matrix former into an oil phase, with the pharmaceutical active being present in at least one of the oil phase and the water phase and with the oil phase comprising a vegetable-based edible oil;
   forming an emulsion from the oil phase and the water phase; and
   lyophilizing the emulsion to form a solid lyophilisate, wherein lyophilizing the emulsion includes first snap freezing the emulsion by cooling the emulsion to a freezing temperature below the temperature at which the emulsion is lyophilized.

2. The method of claim 1, which includes comminuting the solid lyophilisate to provide particles or a powder of the encapsulated pharmaceutical active.

3. The method of claim 1 or 2, in which the hydrophilic polymeric matrix former is selected from the group consisting of a polyethylene glycol, polyacrylic acid, polyether, polyacrylamide, polyvinyl alcohol, polyethylene oxide, polyvinylpyrrolidone, polyoxazoline, polymethacrylate, polyethylenimine, polyphosphate based polymer and mixtures of two or more thereof, and/or in which the hydrophobic polymeric matrix former is selected from the group consisting of a cellulose ether, a poly (lactide-co-glycolide) based polymer, and mixtures of two or more thereof.

4. The method of any of claims 1 to 3, which includes employing a water soluble polymer as a surfactant or emulsifying agent for the forming of the emulsion from the oil phase and the water phase.

5. The method of any of claims 1 to 4, which includes employing a cryoprotectant or lyoprotectant in the emulsion, the cryoprotectant or lyoprotectant being selected from the group consisting of a monosaccharide or a disaccharide, and mixtures thereof.

6. The method of any of claims 1 to 5, in which the vegetable-based edible oil is selected from the group consisting of coconut oil, peanut oil, soya bean oil, olive oil, corn oil, apricot oil, castor oil, macadamia oil, cottonseed oil, palm oil, rapeseed oil, safflower oil, sunflower oil, almond oil, hazelnut oil, flaxseed oil, grapeseed oil, sesame oil, rice bran oil, mustard oil and mixtures of two or more thereof.

7. The method of any of claims 1 to 6, in which the emulsion is lyophilized at a temperature of between -50°C and -70°C and/or in which the emulsion is lyophilized at a pressure of between 7.5 Pa(absolute) and 33.5 Pa(absolute) and/or in which the emulsion is lyophilized for a period of between 24 hours and 120 hours.

8. The method of any of claims 1 to 7, in which the pharmaceutical active is water soluble or hydrophilic and is present in the water phase prior to forming of the emulsion, and/or in which the pharmaceutical active is water-insoluble or hydrophobic and is present in the oil phase prior to forming of the emulsion.

9. The method of claim 2 and claim 4, in which the matrix formers and the vegetable-based oil and the pharmaceutical active are selected such that the powder of the encapsulated pharmaceutical active has a dispersity $Đ_m$ or polydispersity index from dynamic light scattering of less than 0.95.

10. The method of claim 2 and claim 4, or claim 9, in which the matrix formers and the vegetable-based oil and the pharmaceutical active are selected such that a colloidal dispersion of the powder of the encapsulated pharmaceutical active in an aqueous medium has a zeta potential of at least negative 20.00 mV.

11. A powder produced by the method of claim 2 and claim 4, the powder having a dispersity $Đ_m$ or polydispersity index from dynamic light scattering of less than 0.95, with a colloidal dispersion of the powder in an aqueous medium having a zeta potential of at least negative 20.00 mV.

12. The powder of claim 11, which has an average particle size falling in the nano range, or which has an average particle size falling in the micro range.

13. A pharmaceutical product which includes the powder of claim 11 or claim 12.

**Patentansprüche**

1. Verfahren zum Einkapseln von pharmazeutischem Wirkstoff, bei dem das Verfahren
   Dispergieren von hydrophilem, polymerem Matrixbildner in einer Wasserphase und von hydrophobem, polymerem Matrixbildner in einer Ölphase, wobei der pharmazeutische Wirkstoff in mindestens einer von der Ölphase und der Wasserphase vorliegt und die Ölphase ein auf Pflanzen basierendes, essbares Öl ist,
   Bilden einer Emulsion aus der Ölphase und der Wasserphase, und
   Lyophilisieren der Emulsion umfasst, um ein festes Lyophilisat zu bilden, wobei das Lyophilisieren der Emulsion zuerst Schockgefrieren der Emulsion durch Kühlen der Emulsion auf eine Gefriertemperatur unterhalb der Temperatur umfasst, bei der die Emulsion lyophilisiert wird.

2. Verfahren nach Anspruch 1, das Zerkleinern des festen Lyophilisats umfasst, um Teilchen oder ein Pulver des eingekapselten, pharmazeutischen Wirkstoff bereitzustellen.

3. Verfahren nach Anspruch 1 oder 2, bei dem der hydrophile, polymere Matrixbildner aus der Gruppe bestehend aus Polyethylenglykol, Polyacrylsäure, Polyether, Polyacrylamid, Polyvinylalkohol, Polyethylenoxid, Polyvinylpyrrolidon, Polyoxazolin, Polymethacrylat, Polyethylenimin, Polyphosphat-basiertem Polymer und Mischungen von zwei oder mehr derselben ausgewählt ist und/oder bei dem der hydrophobe, polymere Matrixbildner aus der Gruppe bestehend aus Celluloseether, Poly(lactid-co-glycolid)-basiertem Polymer und Mischungen von zwei oder mehr derselben ausgewählt ist.

4. Verfahren nach einem der Ansprüche 1 bis 3, das Verwenden von wasserlöslichem Polymer als Tensid oder Emulgiermittel für die Bildung der Emulsion aus der Ölphase und der Wasserphase umfasst.

5. Verfahren nach einem der Ansprüche 1 bis 4, das Verwenden von Gefrierschutzmittel oder Lyoschutzmittel in der Emulsion umfasst, wobei das Gefrierschutzmittel oder Lyoschutzmittel aus der Gruppe bestehend aus Monosaccharid oder Disaccharid und Mischungen derselben ausgewählt ist.

6. Verfahren nach einem der Ansprüche 1 bis 5, bei dem das auf Pflanzen basierende, essbare Öl aus der Gruppe bestehend aus Kokosnussöl, Erdnussöl, Sojabohnenöl, Olivenöl, Maisöl, Aprikosenöl, Castoröl, Macademiaöl, Baumwollsamenöl, Palmöl, Rapsöl, Distelöl, Sonnenblumenöl, Mandelöl, Haselnussöl, Leinsamenöl, Traubenkernöl, Sesamöl, Reiskleieöl, Senföl und Mischungen von zwei oder mehr derselben ausgewählt ist.

7. Verfahren nach einem der Ansprüche 1 bis 6, bei dem die Emulsion bei einer Temperatur von -50°C bis -70°C lyophilisiert wird und/oder bei dem die Emulsion bei einem Druck von 7,5 Pa (absolut) bis 33.5 Pa (absolut) lyophilisiert wird und/oder bei dem die Emulsion über einen Zeitraum von 24 Stunden bis 120 Stunden lyophilisiert wird.

8. Verfahren nach einem der Ansprüche 1 bis 7, bei dem der pharmazeutische Wirkstoff wasserlöslich oder hydrophil ist und vor dem Bilden der Emulsion in der Wasserphase vorliegt und/oder bei dem der pharmazeutische Wirkstoff wasserunlöslich oder hydrophob ist und vor dem Bilden der Emulsion in der Ölphase vorliegt.

9. Verfahren nach Anspruch 2 und Anspruch 4, bei dem die Matrixbildner und das auf Pflanzen basierende Öl und der pharmazeutische Wirkstoff so ausgewählt sind, dass das Pulver des eingekapselten pharmazeutischen Wirkstoffs eine Dispersität $D_m$ oder einen Polydispersitätsindex gemäß dynamischer Lichtstreuung von unter 0,95 aufweist.

10. Verfahren nach Anspruch 2 und Anspruch 4, oder Anspruch 9, bei dem die Matrixbildner und das auf Pflanzen basierende Öl und der pharmazeutische Wirkstoff so ausgewählt sind, dass eine kolloidale Dispersion des Pulvers des eingekapselten, pharmazeutischen Wirkstoffs in einem wässrigen Medium ein zeta-Potential von mindestens -20,00 mV aufweist.

11. Pulver, das nach dem Verfahren gemäß Anspruch 2 und Anspruch 4 hergestellt worden ist, bei dem das Pulver eine Dispersität $D_m$ oder einen Polydispersitätsindex gemäß dynamischer Lichtstreuung von unter 0,95 aufweist, wobei eine kolloidale Dispersion des Pulvers des eingekapselten, pharmazeutischen Wirkstoffs in einem wässrigen Medium ein zeta-Potential von mindestens -20,00 mV aufweist.

12. Pulver nach Anspruch 11, das eine durchschnittliche Teilchengröße aufweist, die in den Nanobereich fällt, oder das eine durchschnittliche Teilchengröße aufweist, die in den Mikrobereich fällt.

13. Pharmazeutisches Produkt, das das Pulver gemäß Anspruch 11 oder Anspruch 12 enthält.

## Revendications

1. Procédé pour encapsuler un principe actif pharmaceutique, le procédé incluant
la dispersion d'un générateur de matrice polymérique hydrophile dans une phase aqueuse et d'un générateur de matrice polymérique hydrophobe dans une phase huileuse, le principe actif pharmaceutique étant présent dans au moins l'une de la phase huileuse et de la phase aqueuse et la phase huileuse comprenant une huile comestible à base végétale ;
la formation d'une émulsion à partir de la phase huileuse et de la phase aqueuse ; et
la lyophilisation de l'émulsion pour former un lyophilisat solide, où la lyophilisation de l'émulsion inclut d'abord la congélation ultrarapide de l'émulsion par refroidissement de l'émulsion jusqu'à une température de congélation inférieure à la température à laquelle l'émulsion est lyophilisée.

2. Procédé selon la revendication 1, qui inclut la fragmentation du lyophilisat solide pour produire des particules ou une poudre du principe actif pharmaceutique encapsulé.

3. Procédé selon la revendication 1 ou 2, dans lequel le générateur de matrice polymérique hydrophile est choisi dans le groupe consistant en un polyéthylèneglycol, un poly(acide acrylique), un polyéther, un polyacrylamide, un poly(alcool vinylique), un poly(oxyde d'éthylène), une polyvinylpyrrolidone, une polyoxazoline, un polyméthacrylate, une polyéthylèneimine, un polymère à base de polyphosphate et les mélanges de deux ou plusieurs de ceux-ci, et/ou dans lequel le générateur de matrice polymérique hydrophobe est choisi dans le groupe consistant en un éther de cellulose, un polymère à base de poly(lactide-co-glycolide), et les mélanges de deux ou plusieurs de ceux-ci.

4. Procédé selon l'une quelconque des revendications 1 à 3, qui inclut l'emploi d'un polymère soluble dans l'eau comme tensioactif ou agent émulsifiant pour la formation de l'émulsion à partir de la phase huileuse et de la phase aqueuse.

5. Procédé selon l'une quelconque des revendications 1 à 4, qui inclut l'emploi d'un cryoprotecteur ou lyoprotecteur dans l'émulsion, le cryoprotecteur ou lyoprotecteur étant choisi dans le groupe consistant en un monosaccharide ou un disaccharide, et les mélanges de ceux-ci.

6. Procédé selon l'une quelconque des revendications 1 à 5, dans lequel l'huile comestible à base végétale est choisie dans le groupe consistant en l'huile de coco, l'huile d'arachide, l'huile de soja, l'huile d'olive, l'huile de maïs, l'huile d'abricot, l'huile de ricin, l'huile de macadamia, l'huile de graines de coton, l'huile de palme, l'huile de colza, l'huile de carthame, l'huile de tournesol, l'huile d'amande, l'huile de noisette, l'huile de graines de lin, l'huile de pépins de raisin, l'huile de sésame, l'huile de son de riz, l'huile de moutarde et les mélanges de deux ou plusieurs de celles-ci.

7. Procédé selon l'une quelconque des revendications 1 à 6, dans lequel l'émulsion est lyophilisée à une température entre -50°C et -70°C et/ou dans lequel l'émulsion est lyophilisée à une pression entre 7,5 Pa (absolus) et 33,5 Pa (absolus) et/ou dans lequel l'émulsion est lyophilisée pendant une durée entre 24 heures et 120 heures.

8. Procédé selon l'une quelconque des revendications 1 à 7, dans lequel le principe actif pharmaceutique est soluble dans l'eau ou hydrophile et est présent dans la phase aqueuse avant la formation de l'émulsion, et/ou dans lequel le principe actif pharmaceutique est insoluble dans l'eau ou hydrophobe et est présent dans la phase huileuse avant la formation de l'émulsion.

**9.** Procédé selon la revendication 2 et la revendication 4, dans lequel les générateurs de matrice et l'huile à base végétale et le principe actif pharmaceutique sont choisis de telle sorte que la poudre du principe actif pharmaceutique encapsulé a une dispersité $D_m$ ou un indice de polydispersité d'après la diffusion dynamique de la lumière inférieur(e) à 0,95.

**10.** Procédé selon la revendication 2 et la revendication 4, ou la revendication 9, dans lequel les générateurs de matrice et l'huile à base végétale et le principe actif pharmaceutique sont choisis de telle sorte qu'une dispersion colloïdale de la poudre du principe actif pharmaceutique encapsulé dans un milieu aqueux a un potentiel zêta d'au moins 20,00 mV négatifs.

**11.** Poudre produite par le procédé selon la revendication 2 et la revendication 4, la poudre ayant une dispersité $D_m$ ou un indice de polydispersité d'après la diffusion dynamique de la lumière inférieur(e) à 0,95, une dispersion colloïdale de la poudre dans un milieu aqueux ayant un potentiel zêta d'au moins 20,00 mV négatifs.

**12.** Poudre selon la revendication 11, qui a une dimension de particule moyenne située dans la gamme des nanomètres, ou qui a une dimension de particule moyenne située dans la gamme des micromètres.

**13.** Produit pharmaceutique qui inclut la poudre selon la revendication 11 ou la revendication 12.

Fig 1

Fig 2

Fig 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9217162 A1

- EP 2082750 A1

**Non-patent literature cited in the description**

- **DAN YANG YING et al.** Microencapsulated Lactobacillus rhamnosus GG Powders: Relationship of Powder Physical Properties to Probiotic Survival during Storage. *Journal of Food Science,* November 2010, vol. 75 (9

- An enteric-coated dry emulsion formulation for oral insulin delivery. **TOORISAKA E et al.** Journal of Controlled Release. Elsevier, 20 September 2005, vol. 107